# EUROPEAN PATENT SPECIFICATION

(11) **EP 0 936 207 B1**
(45) Date of publication and mention of the grant of the patent: **05.06.2002**
(21) Application number: 99300826.7
(22) Date of filing: 04.02.1999
(51) Int. Cl.: C07C 13/04, C07C 1/20, C07C 33/05

(54) **Process for the preparation of cyclopropylacetylene derivatives**
Verfahren zur Herstellung von Cyclopropylacetylenderivaten
Procédé pour la préparation de dérivés de cyclopropylacétylène

(30) Priority: 06.02.1998 JP 2555898; 19.03.1998 JP 6999298
(43) Date of publication of application: 18.08.1999
(73) Proprietor: KURARAY CO., LTD., Kurashiki-City Okayama Prefecture 710 (JP)
(72) Inventor: Asanuma, Goro, Kurashiki-City, Okayama-Pref. (JP); Takaki, Kazuya, Kurashiki-City, Okayama-Pref. (JP); Ohzono, Shigeo, Kurashiki-City, Okayama-Pref. (JP); Shiono, Manzo, Kurashiki-City, Okayama-Pref. (JP)
(74) Representative: Nachshen, Neil Jacob

(56) References cited:
- THOMPSON A S ET AL: "Use of an Ephedrine Alkoxide to Mediate Enantioselective Addition of an Acetylide to a Prochiral Ketone: Asymmetric Synthesis of the Reverse Transcriptase Inhibitor L-743, 726" TETRAHEDRON LETTERS, vol. 36, no. 49, 4 December 1995 (1995-12-04), page 8937-8940 XP004026777
- SCHOBERTH W ET AL: "Eine einfache Herstellungsmethode für Cyclopropylacetylen" SYNTHESIS, no. 12, 1 December 1972 (1972-12-01), page 703 XP000565549
- I. E. DOLGII ET AL: "Synthesis of Cyclopropylethynylfulvene - The first acetylenic Fulvene" BULLETIN OF THE ACADEMY OF SCIENCES OF THE USSR. DIVISION OF CHEMICAL SCIENCE., vol. 34, 1985, pages 1985-1986, XP002099849 NEW YORK US
- O. M. NEFEDOV ET AL: "Synthesis and Conversions of Cyclopropylacetylenes" BULLETIN OF THE ACADEMY OF SCIENCES OF THE USSR. DIVISION OF CHEMICAL SCIENCE., vol. 27, 1978, pages 1164-1168, XP002099850 NEW YORK US

## Description

### BACKGROUND OF THE INVENTION

### Field of the Invention

The present invention relates to a process for the preparation of a cyclopropylacetylene derivative, an intermediate in synthesis of the cyclopropylacetylene derivative and a process for the preparation of the same. A cyclopropylacetylene derivative produced according to the present invention is useful as an intermediate in synthesis for a compound having a cyclopropane skeleton, for example a benzoxazinone derivative (L-743726), which has an anti-HIV activity (Tetrahedron Letters, vol. 36, p. 8937 (1995)).

### Description of the Related Art

Recently, a large number of physiologically active substances having a cyclopropane skeleton have been discovered. Examples of known methods of producing cyclopropylacetylene, which are useful as an intermediate in synthesis for these compounds, are: (1) a method in which 5-chloropentyne is reacted with n-butyllithium (Tetrahedron Letters, vol. 36, p. 8937 (1995)); and (2) a method in which cyclopropyl methyl ketone is reacted with phosphorus pentachloride in carbon tetrachloride to produce 1,1-dichloro-1-cyclopropylethane, which is subsequently dehydrochlorinated by potassium tert-butoxide (Synthesis, p. 703 (1972)).

However, the method (1) provides a low yield of the raw material 5-chloropentyne as 57% (Journal of American Chemical Society, vol. 67, p. 484 (1945)), and the method (2) gives many by-products and hence is low in the yield of the target product. Therefore, these methods are hard to evaluate as being industrially useful methods for preparing cyclopropylacetylene.

On the other hand, there is a further method, in which a propynol derivative is reacted with an alkyl (di)halide in liquid ammonia in the presence of lithium amide to give an acetylene derivative (Bulletin de la Societe Chimique de France, pp. 201-204 (1968)), but no method for transforming the obtained acetylene derivative to a compound having a cyclopropane skeleton is disclosed or suggested therein. In addition, there is known a method in which cyclopropylacetylene is reacted with acetone or cyclopropyl methyl ketone by ethynylation to give 2-methyl-4-cyclopropyl-3-butyn-2-ol or 2,4-dicyclopropyl-3-butyn-2-ol (Izvetiya Akademii Nark SSSR, Seriya Khimicheskaya, pp. 1339-1344 (1978)), but no description is found for its reverse reaction.

It is, therefore, an aim of the present invention to provide a method by which a cyclopropylacetylene derivative can be produced in a good yield and advantageously in a commercial production.

Another aim of the present invention is to provide an intermediate, which is useful in the production of the cyclopropylacetylene derivative, and a method for the preparation of the intermediate.

### SUMMARY OF THE INVENTION

To be more specific, the present invention provides, in a first aspect, a process for the preparation of a cyclopropylacetylene derivative represented by the following formula (V): 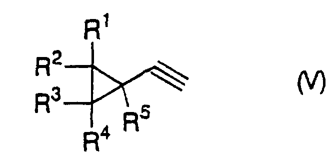 wherein each of R¹, R², R³, R⁴ and R⁵ independently represents a hydrogen atom, an alkyl group, an alkenyl group, an aryl group or an aralkyl group each of which may have a substituent (hereinafter simply referred to as cyclopropylacetylene derivative (V)), which comprises the step of subjecting a cyclopropylpropynol derivative presented by the following formula (IV): 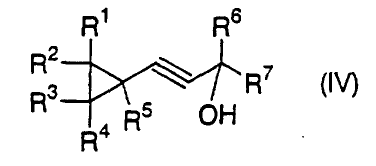 wherein R¹, R², R³, R⁴ and R⁵ have the same meanings as defined above and each of R⁶ and R⁷ represents a hydrogen atom, or an alkyl group, an alkenyl group, an aryl group or an aralkyl group each of which may have a substituent, or R⁶ and R⁷ may together form a ring such as a cycloalkyl group (hereinafter simply referred to as cyclopropylpropynol derivative (IV)), to retro-ethynylation.

In a second aspect the present invention provides a process for the preparation of a cyclopropylacetylene derivative (V), which comprises the steps of reacting a propynol derivative represented by the following formula (I): 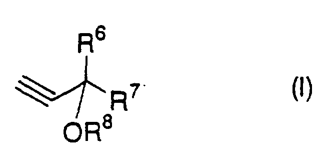 wherein R⁶ and R⁷ have the same meanings as defined above and R⁸ represents a protecting group for a hydroxyl group (hereinafter simply referred to as propynol derivative (I)) with a propane derivative represented by the following formula (VI): 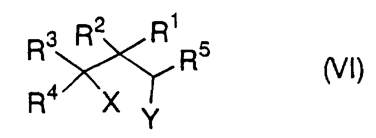 wherein R¹, R², R³, R⁴ and R⁵ have the same meanings as defined above, and each of X and Y represents a leaving group (hereinafter simply referred to as propane derivative (VI)), in the presence of a base in an amount of less than 2 equivalents relative to the propynol derivative (I) at a temperature of 0°C or lower to give an acetylene derivative represented by the following formula (II): 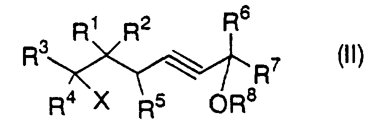 wherein R¹, R², R³, R⁴, R⁵, R⁶, R⁷, R⁸ and X have the same meanings as defined above (hereinafter simply referred to as acetylene derivative (II)), reacting the obtained acetylene derivative (II) with a base to give a cyclopropane derivative represented by the following formula (III): 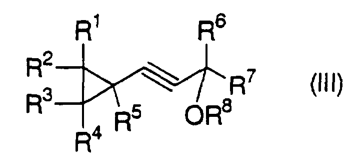 wherein R¹, R², R³, R⁴, R⁵, R⁶, R⁷ and R⁸ have the same meanings as defined above (hereinafter simply referred to as cyclopropane derivative (III)), deprotecting the protecting group for the hydroxyl group of the obtained cyclopropane derivative (III) to give a cyclopropylpropynol derivative (IV), and subjecting the obtained cyclopropylpropynol derivative (IV) to retro-ethynylation.

The present invention provides in a third aspect a process for the preparation of a cyclopropylacetylene derivative (V), which comprises the steps of reacting a propynol derivative (I) with a propane derivative (VI) in the presence of a base in an amount of 2 or more equivalents relative to the propynol derivative (I) to give a cyclopropane derivative (III), deprotecting the protecting group for the hydroxyl group of the obtained cyclopropane derivative (III) to give a cyclopropylpropynol derivative (IV), and subjecting the obtained cyclopropylpropynol derivative (IV) to retro-ethynylation.

In addition and advantageously, the present invention provides in a fourth aspect a cyclopropane derivative represented by the following formula (III-1): 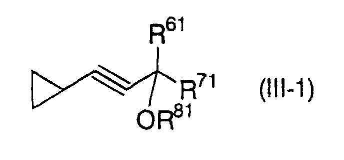 wherein R⁶¹ represents an alkyl group, R⁷¹ represents an alkyl group having 2 or more carbon atoms when R⁶¹ is a methyl group or R⁷¹ represents an alkyl group when R⁶¹ is an alkyl group having 2 or more carbon atoms, and R⁸¹ represents a hydrogen atom or a protecting group for a hydroxyl group.

### DETAILED DESCRIPTION OF THE INVENTION

Unless specified to the contrary the following definitions apply herein with respect to the groups R¹ to R⁸, R⁶¹, R⁷¹ and R⁸¹. The term "alkyl" includes linear and branched alkyl groups of from 1 to 6 carbon atoms which may optionally be substituted by hydroxy, alkoxy or substituted alkoxy groups. The term "aryl" is used to include aryl groups of from 6 to 10 carbon atoms. The term "aralkyl group" is used incorporating the definitions of "alkyl" and "aryl" given above. The term "cycloalkyl" is used to include groups containing from 5 to 8 carbon atoms.

Examples of the alkyl groups represented by R¹, R², R³, R⁴, R⁵, R⁶ and R⁷ in the above formulae include methyl group, ethyl group, propyl group, isopropyl group, butyl group, isobutyl group, tert-butyl group, 4-methylpentyl group and others.

These alkyl groups each may have a substituent and examples of such substituents include hydroxyl group; methoxy group, ethoxy group, propoxy group, butoxy group and other alkoxyl groups; tert-butyldimethylsilyloxy group, tert-butyldiphenylsilyloxy group and other tri-substituted silyloxy groups; and phenyl group, p-methoxyphenyl group, p-chlorophenyl group and other aryl groups.

The alkyl groups represented by R⁶¹ and R⁷¹ include, for instance, methyl group, ethyl group, propyl group, isopropyl group, butyl group, isobutyl group, tert-butyl group, 4-methylpentyl group and others.

Examples of the alkenyl groups represented by R¹, R², R³, R⁴, R⁵, R⁶ and R⁷ include vinyl group, propenyl group and butenyl group; the aryl groups include phenyl group and naphthyl group, for example; and the aralkyl groups include benzyl group, for instance. These alkenyl groups, aryl groups and aralkyl groups each may have a substituent, and examples of such substituents include hydroxyl group; methyl group, ethyl group, propyl group, butyl group and other alkyl groups; methoxy group, ethoxy group, propoxy group, butoxy group and other alkoxyl groups; tert-butyldimethylsilyloxy group, tert-butyldiphenylsilyloxy group and other tri-substituted silyloxy groups; and phenyl group, p-methoxyphenyl group, p-chlorophenyl group and other aryl groups.

As examples of the ring which is formed by R⁶ and R⁷ together, there may be mentioned cycloalkyl rings such as cyclopentane ring, cyclohexane ring, cycloheptane ring, cyclooctane ring or the like.

The protecting groups for a hydroxyl group represented by R⁸ and R⁸¹ include, for example, trimethylsilyl group, tert-butyldimethylsilyl group, tert-butyldiphenylsilyl group and other tri-substituted silyl groups; 1-ethoxy-1-ethyl group, tetrahydrofuranyl group, tetrahydropyranyl group and other acetal groups.

Examples of the leaving groups represented by X and Y include chlorine atom, bromine atom, iodine atom and other halogen atoms; methanesulfonyloxy group, ethanesulfonyloxy group, benzenesulfonyloxy group, p-toluenesulfonyloxy group and other organic sulfonyloxy groups.

A production process of the present invention will be described about each step in detail below.

### Step 1: A step of producing a cyclopropylpropynol derivative (IV) by deprotecting a protecting group for a hydroxyl group of a cyclopropane derivative (III)

When the protecting group for a hydroxyl group is a tri-substituted silyl group, a cyclopropylpropynol derivative (IV) can be obtained by a commonly used desilylation method. Such methods include, for example, a method of reacting a cyclopropane derivative (III) with tetrabutylammonium fluoride in tetrahydrofuran, a method of reacting it with hydrofluoric acid in water, a method of reacting it with acetic acid in water or in a mixture solvent of tetrahydrofuran and water, and a method of reacting it with potassium carbonate in methanol. When the protecting group for a hydroxyl group is an acetal group, a cyclopropylpropynol derivative (IV) can be obtained by a commonly used deacetalation method, including for example a method of reacting a cyclopropane derivative (III) with an acid in an alcoholic solvent.

The reaction temperature is preferably within the range from -100°C to 100°C and more preferably from -30°C to 70°C.

Thus obtained cyclopropylpropynol derivative (IV) can be isolated and purified in the usual manner for isolation and purification of organic compounds. By way of illustration, after confirming the completion of the reaction and neutralizing an acid catalyst with a base such as sodium methoxide, the reaction mixture is poured into water, subjected to extraction with an organic solvent such as diethyl ether, ethyl acetate or methylene chloride, and an extract is washed, if necessary, with an aqueous sodium bicarbonate solution, water, a saline solution or the like in order to remove an acidic substance and a water-soluble substance, the extract is further dried with anhydrous sodium sulfate, anhydrous magnesium sulfate or the like and thereafter further concentrated, and the obtained crude product can be, as necessary, purified by distillation, chromatography, recrystallization or the like. Without purification, the crude product may be provided for next reaction.

### Step 2: A step of producing a cyclopropylacetylene derivative (V) by subjecting a cyclopropylpropynol derivative (IV) to retro-ethynylation

As a method for retro-ethynylation of a cyclopropylpropynol derivative (IV), a method of reacting the compound with a base is commonly employed. Examples of the bases used here include lithium hydroxide, sodium hydroxide, potassium hydroxide and other alkali metal hydroxides; magnesium hydroxide and other alkaline earth metal hydroxides; lithium carbonate, sodium carbonate, potassium carbonate and other carbonates; methyllithium, n-butyllithium, sec-butyllithium, tert-butyllithium and other alkyllithium compounds; phenyllithium and other aryllithium compounds; methylmagnesium chloride, ethylmagnesium bromide and other alkylmagnesium halides; lithium amide, sodium amide, potassium amide, lithium diethylamide, lithium diisopropylamide, lithium bistrimethylsilylamide, sodium bistrimethylsilylamide, potassium bistrimethylsilylamide, bromomagnesium diisopropylamide and other metal amides; lithium methoxide, sodium methoxide, potassium methoxide, sodium ethoxide, potassium ethoxide, sodium tert-butoxide, potassium tert-butoxide and other metal alkoxides; sodium hydride, potassium hydride and other alkali metal hydrides and the like. A catalytic amount of the base is sufficient to use, whereas the amount of the base is preferably within the range from 0.001 equivalent to 5 equivalents relative to the cyclopropylpropynol derivative (IV).

A reaction may generally be conducted in the presence or in the absence of a solvent. The solvent is not specially restricted as far as it does not adversely affect the reaction and includes, for instance, methanol, ethanol, propanol, isopropanol, butanol, sec-butanol, tert-butanol, n-octanol and other alcohols; diethyl ether, tetrahydrofuran, dimethoxyethane and other ethers; pentane, hexane, heptane, octane, petroleum ether, benzene, toluene, xylene and other hydrocarbons; N,N-dimethylformamide, N-methylpyrrolidone, 1,3-dimethyl-2-imidazolidinone, hexamethylphosphoric triamide and other amides; dimethyl sulfoxide; or mixture solvents of these solvents. The amount of the solvent is preferably within the range from 1 to 200 times as much as the weight of the cyclopropylpropynol derivative (IV).

The reaction is preferably carried out under an inert gas atmosphere. The reaction temperature is preferably within the range from -100°C to 150°C, and more preferably from -30°C to 80°C. The reaction can also be conducted while distilling a cyclopropylacetylene derivative (V) produced during the reaction.

Thus obtained cyclopropylacetylene derivative (V) can be isolated and purified in the usual manner for isolation and purification of organic compounds. By way of example, the reaction mixture is poured into water, subjected to extraction with an organic solvent such as diethyl ether, ethyl acetate or methylene chloride, and an extract is washed, if necessary, with an aqueous sodium bicarbonate solution, water, a saline solution or the like to remove an acidic substance and a water-soluble substance, the extract is further dried with anhydrous sodium sulfate, anhydrous magnesium sulfate or the like and thereafter further concentrated, and the obtained crude product can be, as necessary, purified by distillation, chromatography, recrystallization or the like.

As thus described, the present invention provides a process whereby a cyclopropylacetylene derivative can be obtained in a good yield and advantageously on an industrial scale.

The propynol derivative (I) as a starting material in the above steps can be obtained by introducing a protecting group for a hydroxyl group into a propynol compound represented by the following formula: 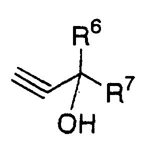 wherein R⁶ and R⁷ have the same meanings as defined above. As such propynol compounds, use can be made of various available compounds, of which preferred are 2-methyl-2-hydroxy-3-butyne, 3-methyl-3-hydroxy-4-propyne, 2,6-dimethyl-6-hydroxy-7-octyne, 2,6-dimethyl-6-hydroxy-oct-2-en-7-yne or the like for their availability on an industrial scale.

The aforementioned reaction of introducing a protecting group for a hydroxyl group into the propynol compound can be carried out in the following manner: For example, a tri-substituted silyl group can be introduced as a protecting group for a hydroxyl group by reacting a tri-substituted silyl halide such as trimethylchlorosilane, tert-butyldimethylchlorosilane or tert-butyldiphenylchlorosilane with the propynol compound in the presence of an organic base such as triethylamine or pyridine. On the other hand, an acetal group as a protecting group for a hydroxyl group can be introduced by reacting a vinyl ether compound such as ethyl vinyl ether, 2,3-dihydrofuran or 3,4-dihydropyran with the propynol compound in the presence of an acid catalyst such as p-toluenesulfonic acid dihydrate, pyridinium p-toluenesulfonate, concentrated sulfuric acid or phosphoric acid.

### EXAMPLES

The present invention will now be described in more detail with reference to the following examples, but these examples should never be construed to limit the scope of the invention.

### EXAMPLE 1

A dried flask (200 ml volume) which had been flushed with nitrogen was cooled to -50°C, gaseous ammonia was then introduced into the cooled flask to give about 100 ml of condensed liquid ammonia, and to the condensed ammonia was added 4.60 g (0.20 mol) of lithium amide. To the mixture were added 7.70 g (0.05 mol) of 2-methyl-2-tetrahydrofuranoxy-3-butyne and 20 ml of tetrahydrofuran at a temperature of -50°C to -40°C, and stirred for 30 minutes at the same temperature. After being added with 9.45 g (0.06 mol) of 1-bromo-3-chloropropane and 10 ml of tetrahydrofuran, the reaction mixture was warmed to 0°C over 10 hours while distilling liquid ammonia off and then stirred at 0°C for 2 hours in order to complete the reaction. After the completion of the reaction, the reaction mixture was poured into 200 ml of an aqueous saturated ammonium chloride solution under ice-cooling for hydrolysis, and then subjected to extraction with two 100 ml portions of diisopropyl ether. The extract was washed with an aqueous saturated sodium bicarbonate solution and a saturated saline solution, dried over anhydrous sodium sulfate and concentrated by distilling off the solvent through a rotary evaporator to give 11.8 g of a crude product.

The crude product was purified by distillation under reduced pressure to give 2-methyl-2-tetrahydrofuranoxy-4-cyclopropyl-3-butyne (9.10 g, purity 97.6%, yield 91.6%) with the following physical data:
Boiling point: 64-66°C/0.2-0.3 Torr
¹H-NMR spectrum (270 MHz, CDCl₃, TMS, ppm) δ : 0.60-0.80 (m, 4H), 1.40 (s, 3H), 1.47 (s, 3H), 1.20-1.30 (m, 1H), 1.70-2.05 (m, 4H), 3.75-4.00 (m, 2H), 5.60-5.70 (m, 1H).

### EXAMPLE 2

A dried flask (200 ml volume) which had been flushed with nitrogen was cooled to -50°C, gaseous ammonia was then introduced into the cooled flask to give about 100 ml of condensed liquid ammonia, and to the ammonia were added 4.60 g (0.20 mol) of lithium amide. To the mixture were added 11.2 g (0.05 mol) of 2,6-dimethyl-6-tetrahydrofuranoxy-7-octyne and 20 ml of tetrahydrofuran at a temperature of -50°C to -40°C, and the mixture was stirred for 30 minutes at the same temperature. After being added with 9.45 g (0.06 mol) of 1-bromo-3-chloropropane and 10 ml of tetrahydrofuran, the reaction mixture was warmed to 0°C over 10 hours while distilling liquid ammonia off and then stirred at 0°C for 2 hours in order to complete the reaction. After the completion of the reaction, the reaction mixture was added to 200 ml of an aqueous saturated ammonium chloride solution under ice-cooling for hydrolysis, and then subjected to extraction with two 100 ml portions of diisopropyl ether. The extract was washed with an aqueous saturated sodium bicarbonate solution and a saturated saline solution, dried over anhydrous sodium sulfate and concentrated by distilling off the solvent through a rotary evaporator to give 14.8 g of a crude product.

The crude product was purified by distillation under reduced pressure to give 2,6-dimethyl-6-tetrahydrofuranoxy-8-cyclopropyl-7-octyne (12.17 g, purity 98.5%, yield 90.8%) having the following physical data:
Boiling point: 84-86°C/0.2-0.3 Torr
¹H-NMR spectrum (270 MHz, CDCl₃, TMS, ppm) δ : 0.60-0.80 (m, 4H), 0.87 (d, 6H, J=6.4 Hz), 1.35 (s), 1.43 (s, 3H together with s of δ =1.35), 1.10-2.05 (m, 12H), 3.75-4.00 (m, 2H), 5.60-5.70 (m, 1H).

### EXAMPLE 3

In a dried flask (300 ml volume) which had been flushed with nitrogen were placed 2,6-dimethyl-6-tetrahydrofuranoxy-8-cyclopropyl-7-octyne (10.72 g, purity 98.5%, 0.04 mol) obtained in Example 2, ethanol (100 ml) and pyridinium p-toluenesulfonate (5.4 mg), and the resulting mixture was heated at 50°C to 60°C for 2 hours. After the reaction was completed, the reaction mixture was cooled to room temperature and to the mixture was added 10 mg of sodium methoxide (25% methanol solution), and ethanol was distilled off through a rotary evaporator. To the obtained concentrate was added 100 ml of water and the resulting mixture was subjected to extraction with two 100 ml portions of ethyl acetate. The extract was washed with an aqueous saturated sodium bicarbonate solution and a saturated saline solution, dried over anhydrous sodium sulfate and consentrated by distilling off the solvent through a rotary evaporator to give 10.1 g of a crude product. The crude product was purified by distillation under reduced pressure to give 2,6-dimethyl-6-hydroxy-8-cyclopropyl-7-octyne (7.33 g, purity 99.0%, yield 93.5%) having the following physical data:
Boiling point: 75-77°C/0.2-0.3 Torr
¹H-NMR spectrum (270 MHz, CDCl₃, TMS, ppm) δ : 0.60-0.80 (m, 4H), 0.89 (d, 6H, J=6.4 Hz), 1.43 (s, 3H), 1.10-1.70 (m, 8H), 2.03 (brs, 1H).

### EXAMPLE 4

In a dried flask (300 ml volume) inside of which had been flushed with nitrogen were placed 2-methyl-2-tetrahydrofuranoxy-4-cyclopropyl-3-butyne (13.4 g, purity 97.5%, 67.3 mmol) obtained in Example 4, ethanol (120 ml) and pyridinium p-toluenesulfonate (6.7 mg), and the resulting mixture was heated at 50°C to 60°C for 1 hour. After the completion of the reaction, the reaction mixture was cooled to room temperature, 10 mg of sodium methoxide (25% methanol solution) was added to the cooled mixture and ethanol was distilled off through a rotary evaporator. The obtained concentrate was added with 100 ml of water and subjected to extraction with two 100 ml portions of ethyl acetate. The extract was washed with an aqueous saturated sodium bicarbonate solution and a saturated saline solution, dried over anhydrous sodium sulfate and consentrated by distilling off the solvent through a rotary evaporator to give 10.8 g of a crude product. The crude product was purified by silica gel column chromatography to give 2-methyl-2-hydroxy-4-cyclopropyl-3-butyne (7.84 g, purity 98.6%, yield 92.6%) having the following physical data:
¹H-NMR spectrum (270 MHz, CDCl₃, TMS, ppm) δ : 0.60-0.80 (m, 4H), 1.15-1.30 (m, 1H), 1.48 (s, 6H), 2.00-2.10 (brs, 1H).

### EXAMPLE 5

In a flask (300 ml volume) were placed 2-methyl-2-hydroxy-4-cyclopropyl-3-butyne (7.60 g, purity 98.6%, 60.4 mmol) obtained in Example 6. octanol (70 ml) and sodium hydroxide (25 mg) and the resulting mixture was heated at an inner temperature of 100°C to 120°C for 2 hours, while distilling off 7.20 g of a mixture of cyclopropylacetylene and acetone produced during the reaction. To the distillate was added 50 ml of heptane and the mixture was washed with water to remove acetone, and the heptane layer was distilled again to give cyclopropylacetylene (3.65 g, purity 99.6%, yield 91.2%).

### EXAMPLE 6

In a flask (300 ml volume) were placed 2,6-dimethyl-6-hydroxy-8-cyclopropyl-7-octyne(7.20 g, purity 99.0%, 36.7 mmol) obtained in Example 5, toluene (70 ml) and sodium hydroxide (29 mg) and the resulting mixture was heated at an inner temperature of 100°C to 120°C for 2 hours, while distilling off 25.0 g of a mixture of toluene and cyclopropylacetylene produced in the reaction. This distillate was distilled again by a rectification apparatus to give cyclopropylacetylene (2.23 g, purity 99.7 %, yield 91.7 %).

## Claims

1. A process for the preparation of a cyclopropylacetylene derivative represented by the following formula (V): 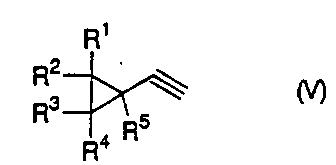 wherein each of R¹, R², R³, R⁴ and R⁵ independently represents a hydrogen atom, an alkyl group, an alkenyl group, an aryl group or an aralkyl group each of which may have a substituent, which comprises the step of subjecting a cyclopropylpropynol derivative represented by the following formula (IV): 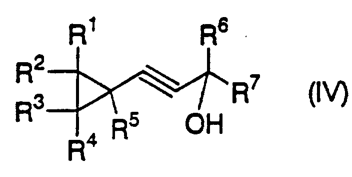 wherein R¹, R², R³, R⁴ and R⁵ have the same meanings as defined above, and each of R⁶ and R⁷ represents a hydrogen atom, or an alkyl group, an alkenyl group, an aryl group or an aralkyl group each of which may have a substituent, or R⁶ and R⁷ may together form a ring, to retro-ethynylation.

2. A process according to claim 1, wherein the cyclopropylpropynol derivative (IV) is prepared by:
reacting a propynol derivative represented by the following formula (I): 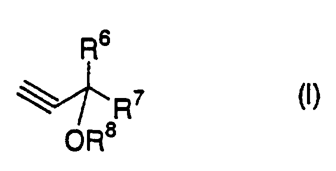 wherein each of R⁶ and R⁷ represents a hydrogen atom, or an alkyl group, an alkenyl group, an aryl group or an aralkyl group each of which may have a substituent, or R⁶ and R⁷ may together form a ring, and R⁸ represents a protecting group for a hydroxyl group, with a propane derivative represented by the following formula (VI): 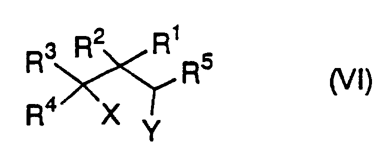 wherein R¹, R², R³, R⁴ and R⁵ have the same meanings as defined in claim 1, and each of X and Y represents a leaving group, in the presence of a base in an amount of less than 2 equivalents relative to the propynol derivative at a temperature of 0°C or lower to give an acetylene derivative represented by the following formula (II): 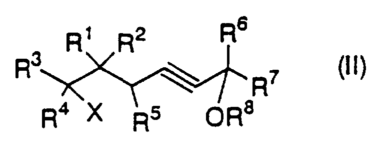 wherein R¹, R², R³, R⁴, R⁵, R⁶, R⁷, R⁸ and X have the same meanings as defined above,
reacting said acetylene derivative with a base to give a cyclopropane derivative represented by the following formula (III) 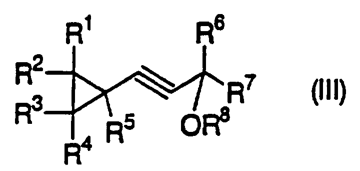 wherein R¹, R², R³, R⁴, R⁵, R⁶, R⁷ and R⁸ have the same meanings as defined above,
and deprotecting the protecting group for the hydroxyl group of said cyclopropane derivative to give the cyclopropylpropynol derivative (IV).

3. A process according to claim 1, wherein the cyclopropylpropynol derivative (IV) is prepared by:
reacting a propynol derivative represented by the following formula (I): 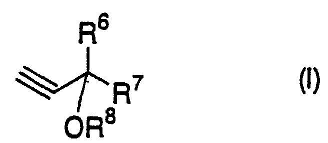 wherein each of R⁶ and R⁷ represents a hydrogen atom, an alkyl group, an alkenyl group, an aryl group or an aralkyl group each of which may have a substituent, or R⁶ and R⁷ may together form a ring, and R⁸ represents a protecting group for a hydroxyl group, with a propane derivative represented by the following formula (VI): 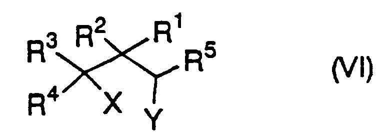 wherein R¹, R², R³, R⁴ and R⁵ have the same meanings as defined in claim 1, and each of X and Y represents a leaving group, in the presence of a base in an amount of 2 or more equivalents relative to the propynol derivative to give a cyclopropane derivative represented by the following formula (III): 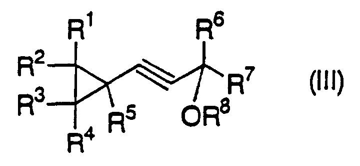 wherein R¹, R², R³, R⁴, R⁵, R⁶, R⁷ and R⁸ have the same meanings as defined above,
deprotecting the protecting group for the hydroxyl group of said cyclopropane derivative to give the cyclopropylpropynol derivative (IV).

4. A cyclopropane derivative represented by the following formula (III-1): 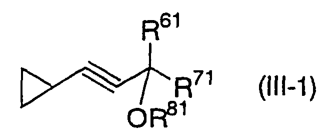 wherein R⁶¹ represents an alkyl group, R⁷¹ represents an alkyl group having 2 or more carbon atoms when R⁶¹ is a methyl group, or R⁷¹ represents an alkyl group when R⁶¹ is an alkyl group having 2 or more carbon atoms, and R⁸¹ represents a hydrogen atom or a protecting group for a hydroxyl group.

5. The cyclopropane derivative as claimed in Claim 4 wherein R⁶¹ is methyl group, R⁷¹ is 4-methylpentyl group and R⁸¹ is a protecting group for a hydroxyl group in the formula (III-1).

## Patentansprüche

1. Verfahren zur Herstellung eines durch die folgende Formal (V) dargestellten Cyclopropylacetylenderivats: 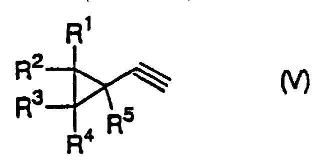 wobei jeder von R¹, R², R³, R⁴ und R⁵ unabhängig voneinander ein Wasserstoffatom, einen Alkylrest, einen Alkenylrest, einen Arylrest oder einen Aralkylrest darstellt, wobei jeder der Reste einen Substituenten aufweisen kann, welches den Schritt des Unterwerfens einer durch die folgende Formel (IV) dargestellten Cyclopropylpropinolderivats: 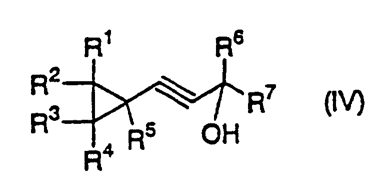 wobei R¹, R², R³, R⁴ und R⁵ dieselben wie vorstehend definierten Bedeutungen haben und jeder von R⁶ und R⁷ ein Wasserstoffatom darstellt, oder einen Alkylrest, einen Alkenylrest, einen Arylrest oder einen Aralkylrest, wobei jeder der Reste einen Substituenten aufweisen kann, oder R⁶ und R⁷ zusammen einen Ring bilden können, der Retro-Ethinylierung umfaßt.

2. Verfahren nach Anspruch 1, wobei das Cyclopropylpropinolderivat (IV) hergestellt wird durch:
Umsetzen eines durch die folgende Formel (I) dargestellten Propinolderivats: 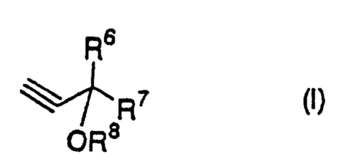 wobei jeder von R⁶ und R⁷ ein Wasserstoffatom darstellt, oder einen Alkylrest, einen Alkenylrest, einen Arylrest oder einen Aralkylrest, wobei jeder der Reste einen Substituenten aufweisen kann, oder R⁶ und R⁷ zusammen einen Ring bilden können, und R⁸ eine Schutzgruppe für eine Hydroxylgruppe darstellt, mit einem durch die folgende Formel (VI) dargestellten Propanderivat: 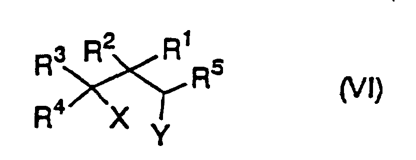 wobei R¹, R², R³, R⁴ und R⁵ dieselben wie in Anspruch 1 definierten Bedeutungen haben, und X und Y jeweils eine Austrittsgruppe darstellen, in Gegenwart einer Base in einer Menge von weniger als zwei Äquivalenten, bezogen auf das Propinolderivat, bei einer Temperatur von 0°C oder darunter, um ein durch die folgende Formel (II) dargestelltes Acetylenderivats zu bilden: 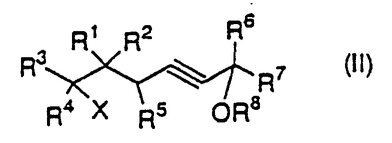 wobei R¹, R², R³, R⁴, R⁵, R⁶, R⁷, R⁸ und X dieselben wie vorstehend definierten Bedeutungen haben,
Umsetzen des Acetylenderivats mit einer Base, um ein durch die nachstehende Formel (III) dargestelltes Cyclopropanderivat zu bilden: 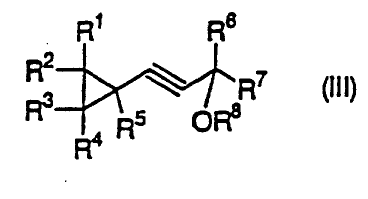 wobei R¹, R², R³, R⁴, R⁵, R⁶, R⁷ und R⁸ dieselben wie vorstehend definierten Bedeutungen haben,
und Entfernen der Schutzgruppe für die Hydroxylgruppe des Cyclopropanderivats, um das Cyclopropylpropinolderivat (IV) zu bilden.

3. Verfahren nach Anspruch 1, wobei das Cyclopropylpropinolderivat (IV) hergestellt wird durch:
Umsetzen eines durch die folgende Formel (I) dargestellten Propinolderivats: 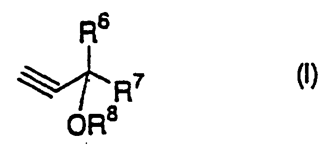 wobei jeder von R⁶ und R⁷ ein Wasserstoffatom, einen Alkylrest, einen Alkenylrest, einen Arylrest oder einen Aralkylrest darstellt, wobei jeder der Reste einen Substituenten aufweisen kann, oder R⁶ und R⁷ zusammen einen Ring bilden können und R⁸ eine Schutzgruppe für eine Hydroxylgruppe darstellt, mit einem durch die folgende Formel (VI) dargestellten Propanderivat: 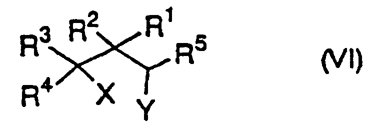 wobei R¹, R², R³, R⁴ und R⁵ dieselben wie in Anspruch 1 definierten Bedeutungen haben, und X und Y jeweils eine Austrittsgruppe darstellen, in Gegenwart einer Base in einer Menge von 2 oder mehr Äquivalenten, bezogen auf das Propinolderivat, um ein durch die folgende Formel (III) dargestelltes Cyclopropanderivat zu bilden: 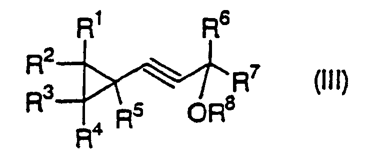 wobei R¹, R², R³, R⁴, R⁵, R⁶, R⁷ und R⁸ dieselben wie vorstehend definierten Bedeutungen haben,
Entfernen der Schutzgruppe für die Hydroxylgruppe des Cyclopropanderivats, um das Cyclopropylpropinolderivat (IV) zu bilden.

4. Cyclopropanderivat, dargestellt durch die folgende Formel (III-1): 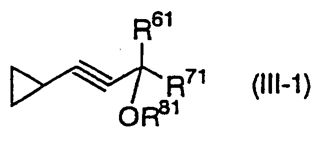 wobei R⁶¹ einen Alkylrest darstellt, R⁷¹ einen Alkylrest mit zwei oder mehr Kohlenstoffatomen darstellt, wenn R⁶¹ eine Methylgruppe ist, oder R⁷¹ einen Alkylrest darstellt, wenn R⁶¹ einen Alkylrest mit zwei oder mehr Kohlenstoffatomen darstellt, und R⁸¹ ein Wasserstoffatom oder eine Schutzgruppe für eine Hydroxylgruppe darstellt.

5. Cyclopropanderivat nach Anspruch 4, wobei R⁶¹ ein Methylrest ist, R⁷¹ ein 4-Methylpentylrest ist und R⁸¹ eine Schutzgruppe für eine Hydroxylgruppe in der Formel (III-1) ist.

## Revendications

1. Procédé de préparation d'un dérivé de cyclopropylacetylène représenté par la formule (V) suivante : 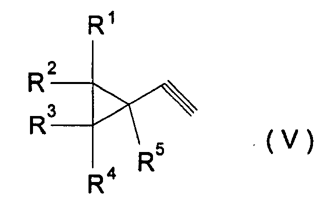 dans laquelle les groupes R¹, R², R³, R⁴, et R⁵ représentent indépendamment chacun un atome d'hydrogène, un groupe alkyle, un groupe alcényle, un groupe aryle ou un groupe aralkyle pouvant être éventuellement tous substitués, qui comprend l'étape consistant à soumettre le dérivé de cyclopropylpropynol représenté par la formule (IV) suivante: 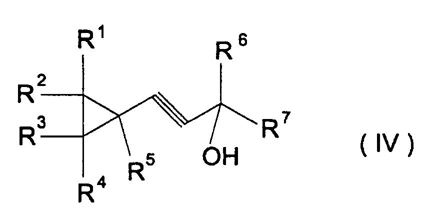 dans laquelle R¹, R², R³, R⁴, et R⁵ ont la même signification que précédemment, et les groupes R⁶, et R⁷ représentent chacun un atome d'hydrogène ou un groupe alkyle, un groupe alcényle, un groupe aryle ou un groupe aralkyle pouvant être tous les deux éventuellement substitués, ou R⁶ et R⁷ peuvent former conjointement un cycle, à une rétroethynylation.

2. Procédé selon le revendication 1, dans lequel le dérivé de cyclopropylpropynol (IV) est préparé par :
la mise en réaction du dérivé de propynol représenté par la formule (I) suivante : 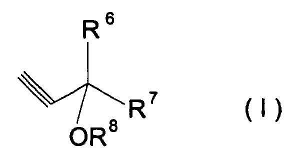 dans laquelle R⁶ et R⁷ représentent chacun un atome d'hydrogène, un groupe alkyle, un groupe alcényle, un groupe aryle ou un groupe aralkyle pouvant être tous les deux éventuellement substitués, ou R⁶ et R⁷ peuvent former conjointement un cycle, et R⁸ représente un groupe protecteur du groupe hydroxyle, avec un dérivé de propane représenté par la formule (IV) suivante: 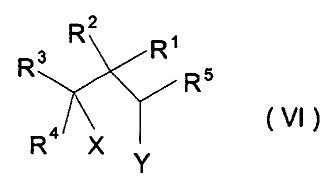 dans laquelle R¹, R², R³, R⁴, et R⁵ sont tels que définis à la revendication 1, et les groupes X et Y représentent chacun un groupe partant, en présence d'une base en proportion inférieure à 2 équivalents par rapport au dérivé de propynol à une température inférieure ou égale à 0°C pour former un dérivé d'acétylène représenté par la formule (II) suivante : 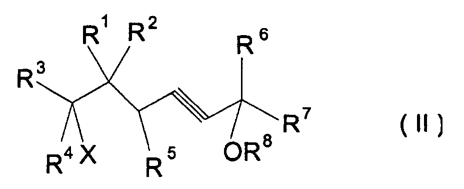 dans laquelle R¹, R², R³, R⁴, R⁵, R⁶, R⁷, R⁸ et X ont la même signification que précédemment,
la mise en réaction dudit dérivé d'acétylène avec une base pour former le dérivé de cyclopropane représenté par la formule (III) suivante : 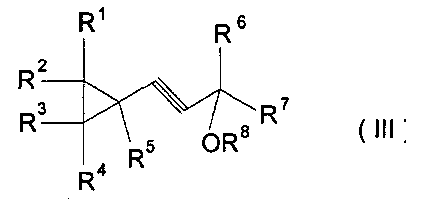 dans laquelle R¹, R², R³, R⁴, R⁵, R⁶, R⁷ et R⁸ ont la même signification que précédemment,
et l'élimination du groupe protecteur du groupe hydroxyle dudit dérivé de cyclopropane pour former le dérivé de cyclopropylpropynol (IV).

3. Un procédé selon le revendication 1, dans lequel le dérivé de cyclopropylpropynol (IV) est préparé par :
la mise en réaction du dérivé de propynol représenté par la formule (I) suivante : 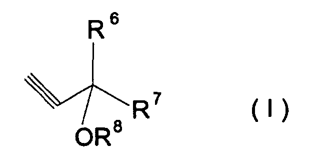 dans laquelle les groupes R⁶ et R⁷ représentent chacun un atome d'hydrogène, un groupe alkyle, un groupe alcényle, un groupe aryle ou un groupe aralkyle pouvant être tous éventuellement substitués, ou R⁶, et R⁷ peuvent former conjointement un cycle, et R⁸ représente un groupe protecteur du groupe hydroxyle, avec un dérivé de propane représenté par la formule (VI) suivante: 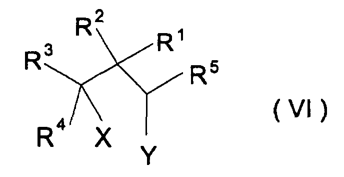 dans laquelle R¹, R², R³, R⁴, et R⁵ sont tels que définis à la revendication 1, et les groupes X et Y représentent chacun un groupe partant, en présence d'une base en proportion supérieure ou égale à 2 équivalents par rapport au dérivé de propynol pour former un dérivé de cyclopropane représenté par la formule (III) suivante : 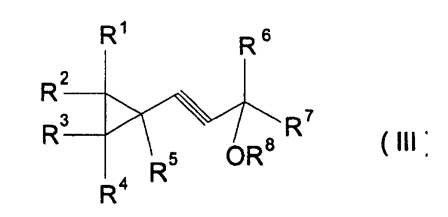 dans laquelle R¹, R², R³, R⁴, R⁵, R⁶, R⁷ et R⁸ ont la même signification que précédemment,
et l'élimination du groupe protecteur du groupe hydroxyle dudit dérivé de cyclopropane pour former le dérivé de cyclopropylpropynol (IV).

4. Dérivé de cyclopropane représenté par la formule (III-1) suivante : 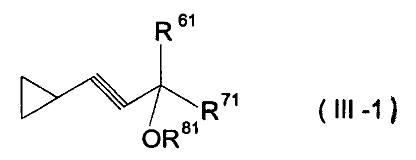 dans laquelle R⁶¹ représente un groupe alkyle, R⁷¹ représente un groupe alkyle ayant 2 atomes de carbone ou plus lorsque R⁶¹ est un groupe méthyle, ou R⁷¹ représente un groupe alkyle lorsque R⁶¹ est un groupe alkyle comportant deux atomes de carbone ou plus, et R⁸¹ représente un atome d'hydrogène ou un groupe protecteur de groupe hydroxyle.

5. Dérivé de cyclopropane selon la revendication 4, dans lequel R⁶¹ est un groupe méthyle, R⁷¹ est un groupe 4-méthylpentyle et R⁸¹ est un groupe protecteur du groupe hydroxyle dans la formule (III-1).
